# EUROPÄISCHE PATENTSCHRIFT

(11) **EP 0 602 380 B1**
(45) Veröffentlichungstag und Bekanntmachung des Hinweises auf die Patenterteilung: **11.09.1996**
(21) Anmeldenummer: 93118010.3
(22) Anmeldetag: 05.11.1993
(51) Int. Cl.: B65D 33/01, A61B 19/02, A61L 11/00, A61L 2/26

(54) **Behälter für die Sterilisation und Desinfektion**
Container for sterilizing or desinfecting
Récipient pour la stérilisation et la désinfection

(30) Priorität: 15.12.1992 DE 9217097 U
(43) Veröffentlichungstag der Anmeldung: 22.06.1994
(73) Patentinhaber: MMM MÜNCHENER MEDIZIN MECHANIK GMBH, D-81369 München (DE)
(72) Erfinder: Goder, Franz, D-81375 München (DE)
(74) Vertreter: Grünecker, Kinkeldey, Stockmair & Schwanhäusser Anwaltssozietät

(56) Entgegenhaltungen:
- EP-A- 0 155 035
- WO-A-91/01927
- DE-U- 9 212 865
- FR-A- 2 603 164
- NL-A- 6 904 549
- US-A- 4 515 841
- US-A- 4 670 227

## Beschreibung

Die Erfindung bezieht sich auf einen verschließbaren sack- bzw. beutelartigen Behälter für infektiösen Abfall, mit flüssigkeits- und gasundurchlässigem Wandmaterial und mindestens einem darin ausgebildeten, für Flüssigkeiten, Gase und Dämpfe durchlässigen Durchlässigkeitsbereich, und einer Einbringöffnung, welche nach dem Einbringen von Abfall geschlossen wird.

In der Desinfektions- bzw. Sterilisiertechnik sind verschließbare Tonnen mit einem Druck-Vakuumventil bekannt, in welche Abfallsäcke eingehängt werden. Nach Einbringen von Abfall werden die Tonnen verschlossen und geschlossen einer Desinfektions- bzw. Sterilisieranlage zugeführt. Nach Abschluß des Desinfektions- bzw. Sterilisierverfahrens werden die Behälter der Anlage entnommen, geöffnet und die eingehängten Säcke entnommen. Die Säcke bleiben im allgemeinen geöffnet und werden einem Preßcontainer oder direkt einer Deponie bzw. einer Verbrennungsanlage zugeführt. In jeden entleerten Behälter wird ein neuer Abfallsack eingehängt und der Behälter wieder an seinen Einfüllort, z.B. einen Operationssaal, zurückgebracht. Es können nicht mehr Säcke gefüllt werden, als Behälter vorhanden sind. Eine direkte Bevorratung der Behälter am Einsatzort bereitet Platzprobleme, durch das Zurückbringen der Behälter können Verunreinigungen durch das Personal oder die Behälter an den Einfüllort, z.B. einen Operationssaal, gelangen.

In der Sterilisiertechnik werden ferner, wie beispielsweise aus DE-U-92 12 865.3 bekannt, Müllsäcke verwendet, welche in ihrem oberen Bereich mit einem Kunststoffvlies-Streifen versehen sind. Diese Müllsäcke werden nach dem Einbringen von Abfall derart geschlossen, daß der Vlies-Streifen weiterhin mit dem Innenraum des Müllsackes in Verbindung steht. Über diesen Vlies-Streifen können, insbesondere bei Verformung des Müllsackes, beispielsweise beim Transport zu einer Desinfektions- bzw. Sterilisieranlage, auch in geschlossenem Zustand des Müllsackes aus dem Sackinneren mit Keimen behaftete Gase und Dämpfe austreten.

Aus US-A-45 15 841 ist ein Müllsack für infektiösen Müll bekannt, welcher aus einem flexiblen Material ausgebildet ist, das unter Einwirkung eines Sterilisierverfahrens Poren bildet.

Des weiteren sind Kunststofftonnen bekannt, welche nach Einbringen von Abfall mit einem durch Hitzeeinwirkung zerstörbaren Deckel verschlossen werden. Bei diesen Tonnen handelt es sich um Einweggebinde, welche nach einmaligem Gebrauch mit Inhalt einer Deponie zugeführt werden. Die Tonnen sind sperrig, in der Herstellung aufwendig und teuer, und sie können nicht in größeren Mengen am Einsatzort bevorratet werden.

Der Erfindung liegt die Aufgabe zugrunde, einen Behälter der eingangs angegebenen Art zu schaffen, welcher einfach und sicher ohne Kontaminationsgefahr für das Bedienungs- bzw. Entsorgungspersonal handhabbar ist, der in gefülltem und geschlossenem Zustand bis zur Durchführung eines Desinfektions- bzw. Sterilisationsverfahrens flüssigkeits-, gas- und dampfdicht ist und umweltfreundlich und wirtschaftlich entsorgt werden kann.

Diese Aufgabe wird erfindungsgemäß durch die in den Patentansprüchen 1, 2 und 18 angegebenen Merkmalskombinationen gelöst.

Dadurch wird es auf vorteilhafte Weise möglich, auf große Mengen von Desinfizier- bzw. Sterilisiergut in einem vollständig verschließbaren Behälter kontaminationssicher zu erfassen und darin zu desinfizieren bzw. zu sterilisieren. Da der Durchlässigkeitsbereich zunächst unwirksam gehalten wird, können keine mit Keimen behafteten Gase oder Dämpfe aus dem Inneren des Behälters austreten. Eine den Behälter handhabende Person wird dadurch wirkungsvoll vor Infektionen geschützt. Der gefüllte und verschlossene Behälter muß nicht umgehend ein Desinfektions- bzw. Sterilisationsanlage zugeführt werden.

Dadurch, daß der Behälter beutelartig mit flexiblem Wandmaterial ausgebildet ist, wird es möglich, den Behälter vor Gebrauch platzsparend zusammenzufalten, so daß eine Vorratshaltung der Behälter direkt am Einsatzort erfolgen kann. Auch bei unvollständigem Füllen des Behälters erweist sich die flexible Wandung als besonders vorteilhaft, da der Behälter bei seiner weiteren Legerung nur das der Müllmenge entsprechende Raumvolumen beansprucht. Der Behälter kann besonders universell in bekannten Sackeinfüllhilfen bzw. Sackeinhängevorrichtungen angebracht werden.

Eine zum Unwirksamhalten des Durchlässigkeitsbereiches besonders günstige Ausgestaltung des Behälters ist dadurch gegeben, daß die Sperreinrichtung in Schließstellung den Behälterinhalt von dem Durchlässigkeitsbereich trennt, insbesondere durch ein Zusammenschnüren des beutelartigen Behälters im Bereich der Einbringöffnung. Dadurch wird ein vollständiger Verschluß des Behälters gewährleistet und eine einfache Ausbildung des Durchlässigkeitsbereiches möglich.

Eine weitere, zum Unwirksamhalten des Durchlässigkeitsbebereiches besonders günstige Ausgestaltung des Behälters ist dadurch gegeben, daß die Sperreinrichtung den mit dem Behälterinhalt in Verbindung stehenden Durchlässigkeitsbereich abdeckt. Dadurch ist es möglich, den Durchlässigkeitsbereich bereits während des Einbringens von Abfall in den Behälter unwirksam zu halten, wodurch sich insbesondere dessen Verschmutzung nicht nachteilig auswirkt.

Eine vorteilhafte Ausbildung des Behälters ist dadurch gegeben, daß mit Eintreten der Veränderung des physikalischen Parameters um den vorbestimmten Betrag die Sperrvorrichtung eine Verbindung zwischen dem Inhalt des Behälters und dessen Umgebung über den Durchlässigkeitsbereich freigibt. Dadurch wird es möglich, daß der Durchlässigkeitsbereich selbsttätig wirksam wird und der Inhalt des Behälters erst nach dem Einbringen in eine Desinfektions- bzw. Sterilisationsanlage mit der Umgebung des Behälters in Verbindung tritt, wodurch ein besonders effektiver Personenschutz gewährleistet ist.

Eine günstige Ausgestaltung des Behälters ist dadurch gegeben, daß eine Verschlußeinrichtung vorgesehen ist, mittels derer der Behälter im Bereich der Einbringöffnung verschließbar ist und während eines Desinfektions- bzw. Sterilisationsverfahrens geschlossen bleibt, da dadurch der Abtransport des desinfizierten bzw. sterilisierten Abfalls ohne ein erneutes Verschließen des Behälters möglich wird. Insbesondere bei einer sackartigen Ausbildung des Behälters kann der behandelte und gefüllte Behälter wie ein üblicher Müllsack gehandhabt werden. Die Verschlußeinrichtung kann insbesondere aus einem mit einer Verdrillvorrichtung gespannten Draht oder einem Kabelbinder aus Kunststoff, z.B. aus Polyamid 6.6 HS bestehen.

Ein besonders vorteilhaftes Verschließen des Behälters ist dadurch gegeben, daß ein Faltenhals bildbar ist und der Durchlässigkeitsbereich im Faltenhals zwischen der Sperrvorrichtung und der Verschlußeinrichtung liegt. Dadurch wird der Durchlässigkeitsbereich von dem Behälterinhalt getrennt und der Behälter bleibt nach Lösen der Sperrvorrichtung weiterhin im Bereich der Einbringöffnung verschlossen. Die Verschlußeinrichtung kann hierbei in einem Verknoten des Faltenhalses bestehen.

Eine günstige konstruktive Ausbildung des Behälters ist dadurch gegeben, daß die Sperreinrichtung in Schließstellung den beutelartigen Behälter durch Zusammenschnüren des Faltenhalses verschlossen hält und sich bei Auftreten eines vorbestimmten Temperatursprunges öffnet, wobei der vormals zusammengeschnürte Bereich des Faltenhalses einen Durchgangsquerschnitt freigibt und eine Verbindung zwischen dem Durchlässigkeitsbereich und dem Behälterinhalt entsteht.

Ein Öffnen der Sperreinrichtung durch Eintreten eines bestimmten Temperatursprunges ist besonders günstig anzuwenden bei einem Desinfektions- bzw. Sterilisierverfahren mittels Erhitzung z.B. mittels Heißdampf. Das Öffnen der Sperreinrichtung tritt dabei ohne zusätzliche Vorkehrungen selbsttätig ein. Die Sperrvorrichtung kann dabei vorteilhafterweise einen Binder bzw. ein Band aus Polyethylen aufweisen.

Eine besonders vorteilhafte Ausbildung des Behälters ist dadurch gegeben, daß die Sperreinrichtung als Schlinge oder Ring ausgebildet ist, welcher mit mindestens einer Sollbruchstelle versehen ist. Eine derartige Einrichtung ist einfach und unter geringen Kosten herstellbar. Neben dem ausschließlich auf das Auftreten von an der Sperrvorrichtung angreifenden Kräften, z.B. durch Aufblähen des Sackes, zurückzuführenden Aufbrechen der Sollbruchstelle kann das Wirksamwerden der Sollbruchstelle von anderen physikalischen Parametern abhängig gemacht werden. So ist es z.B. möglich, in der Sperreinrichtung ein Element mit mindestens einem Hohlraum bzw. einer verschlossenen Kammer vorzusehen, welches sich bei Veränderung des Umgebungsdruckes, insbesondere Druckabsenkung, verformt und das Öffnen der Sperrvorrichtung, z.B. durch Auslenken eines Verriegelungsfederelementes, aus einer Verrastungsstellung einleitet. Es ist auch möglich, durch Eintreten einer chemischen Reaktion die Sollbruchstelle wirksam werden zu lassen. Mit dieser Sperreinrichtung können in vorteilhafter Weise auch übliche Müllsäcke verschlossen werden. Auch diese Säcke können dann in geschlossenem Zustand einem Desinfektionsbzw. Sterilisationsverfahren zugeführt werden.

Eine weitere besonders günstige konstruktive Ausbildung des Behälters ist dadurch gegeben, daß die Sperreinrichtung in Schließstellung den beutelartigen Behälter durch Zusammenschnüren des Faltenhalses derart verschlossen hält, daß eine Verbindung zwischen dem Durchlässigkeitsbereich und dem Inhalt des Behälters zunächst gesperrt ist und sich bei Verformung des Behälters durch Auftreten eines vorbestimmten Überdrucks im Behälterinneren freigegeben wird, so daß der Durchlässigkeitsbereich mit dem Inhalt des Behälters in Verbindung steht. Das Herstellen der Verbindung zwischen dem Durchlässigkeitsbereich und dem Inhalt des Behälters kann dabei insbesondere dadurch erreicht werden, daß durch Verformung des Behälters, insbesondere durch Auftreten von Überdruck im Inneren des Behälters ein Ring bzw. eine Schlinge entlang des Faltenhalses derart verschoben wird, daß der Durchlässigkeitsbereich nicht mehr von dem Behälterinhalt getrennt ist.

Eine besonders vorteilhafte Ausbildung des Behälters ist dadurch gegeben, daß der Durchlässigkeitsbereich durch Perforation bzw. Nadeln, Stanzen und/oder Schneiden des Behälterwandmateriales gebildet ist. Dadurch ist der Durchlässigkeitsbereich zu besonders geringen Kosten herstellbar. Neben dem Nadeln und Stanzen können insbesondere durch das Schneiden günstig geformte Öffnungsquerschnitte erzeugt werden, welche z.B. nach einem Gasdurchtritt durch die Eigenelastizität des Materiales in eine Schließstellung mit wesentlichen kleinerem Öffnungsquerschnitt zurücktreten.

Eine besonders günstige Ausgestaltung des Behälters ist dadurch gegeben, daß der Durchlässigkeitsbereich durch Substitution von Wandmaterial mit mindestens einem flüssigkeits-, gas- und dampfdurchlässigen Element gebildet ist. Dadurch wird es in vorteilhafter Weise möglich, die jeweiligen Eigenschaften des Materiales besonders gezielt zur Wirkung zu bringen. Besonders günstig erweist sich auch, das Material des Durchlässigkeitsbreiches bzw. einen Flächenabschnitt davon mit einem Indikator zu versehen, welcher unter Einwirkung des Desinfektions- bzw. Sterilisationsverfahrens seine Farbe ändert, so daß deutlich erkennbar ist, ob der gefüllte Behälter dem Behandlungsverfahren unterzogen wurde. Des weiteren ist es auch in besonders günstiger Weise möglich, das Material des Durchlässigkeitsbereiches mit keimtötenden Zusätzen zu versehen.

Eine besonders günstige Ausgestaltung des Behälters ist auch dadurch gegeben, daß der Durchlässigkeitsbereich aus einem mindestens zweilagig ausgebildeten folienartigen Element besteht, dessen Lagen mit Durchgangsöffnungen versehen sind, welche derart zueinander versetzt angeordnet sind, daß jede Öffnung zumindest teilweise von einer Folienlage überdeckt wird. Dadurch wird in besonders günstiger Weise möglich, auch bei großen Durchgangsöffnungen eine sichere Zurückhaltewirkung bezüglich des in dem Behälter eingebrachten Abfalls zu erhalten. Eine Berührung mit dem Inhalt des Sackes, insbesondere nach dem Desinfektions- bzw. Sterilisationsverfahren, wird dadurch besonders einfach und wirkungsvoll vermieden. Die Folienlagen können besonders einfach durch Kleben oder Schweißen und insbesondere durch eine Kombination von Kleben und Schweißen aufgebracht werden.

Eine vorteilhafte Ausbildung des Behälters ist dadurch gegeben, daß der Durchlässigkeitsbereich streifenförmig ausgebildet ist. Ein derartig ausgebildeter Durchlässigkeitsbereich ist besonders einfach durch Überrollen mit einer Nadel, Stanz- oder Schneidwalze herzustellen. Dabei ist es auch möglich, mehrere aufeinanderliegende Folienlagen bzw. Säcke gleichzeitig zu bearbeiten. Es ist möglich, bereits mit einem streifenförmigen Durchlässigkeitsbereich einen ausreichenden Durchgangsquerschnitt zu erzeugen.

Eine besonders vorteilhafte Ausbildung des Behälters ist dadurch gegeben, daß die Sperrvorrichtung als auf den Durchlässigkeitsbereich aufgebrachte Folie ausgebildet ist. Eine derartige Sperrvorrichtung ist besonders funktionssicher und komfortabel, da nach Einbringen des Abfalls der Behälter, insbesondere der Sack, mit nur einer Verschlußeinrichtung zu verschließen ist. Es ist möglich, die Folie aus einem wärmeunbeständigen Material zu fertigen, so daß sich die Folie während des Desinfektions- bzw. Sterilisationsverfahrens derart verändert, daß der Durchlässigkeitsbereich freigegeben wird.

Eine besonders günstige Ausgestaltung des Behälters ist dadurch gegeben, daß die Sperrvorrichtung, insbesondere die Folie, auf ihrer den Durchlässigkeitsbereich zugewandten Seite eine Adhäsivmittelschicht aufweist. Eine derartige Sperrvorrichtung ist besonders einfach herstellbar, wirkungsvoll und funktionssicher.

Die vorstehend genannte Sperrvorrichtung ist mit Vorteil erfindungsgemäß so gestaltet, daß die Adhäsivmittelschicht aus einer wärmeunbeständigen Klebeschicht besteht. Neben einem Erweichen bzw. Schmelzen der Klebestelle ist insbesondere auch ein Zersetzen bzw. Verändern der chemischen Struktur des Klebstoffs möglich.

Eine zum Unwirksamhalten des Durchlässigkeitsbereiches besonders günstige Ausgestaltung des Behälters ist dadurch gegeben, daß der Durchlässigkeitsbereich durch Überfalten mit einem undurchlässigen Wandbereich zunächst verdeckt und durch die Sperreinrichtung, vorzugsweise eine Verklebung, mit geringer Temperaturfestigkeit in einer Schließstellung flüssigkeits-, gas- und dampfdicht gehalten ist. Eine derartige Ausbildung ist einfach und unter geringen Kosten ohne zusätzliches Folienmaterial herstellbar. Neben dem Lösen einer Verklebung unter Einwirkung von Wärme wird die Freigabe des Durchlässigkeitsbereiches auch durch das Aufblähen des Sackes unterstützt. Es sind Faltungen möglich, welche Klebestellen mit bestimmten Schubfestigkeiten durch Schälen lösen.

Weitere Einzelheiten und Vorteile ergeben sich aus der nachfolgenden Beschreibung eines Ausführungsbeispieles in Verbindung mit den Zeichnungen. Es zeigen:
- Fig. 1: eine perspektivische Ansicht eines Ausführungsbeispieles des erfindungsgemäßen Behälters in geschlossenem Zustand,
- Fig. 2: eine perspektivische Ansicht des Ausführungsbeispieles des erfindungsgemäßen Behälters gemäß Fig. 1 in einem Zustand, in welchem der Durchlässigkeitsbereich nicht von dem Behälterinhalt getrennt ist,
- Fig. 3: eine perspektivische Ansicht eines Ausschnittes eines zweiten Ausführungsbeispieles aus dem Durchlässigkeitsbereich mit versetzt angeordneten Durchgangsöffnungen,
- Fig. 4: einen unwirksam gehaltenen Durchlässigkeitsbereich gemäß einem dritten Ausführungsbeispiel,
- Fig. 5: einen Ausschnitt eines unwirksam gehaltenen Durchlässigkeitsbereiches gemäß einem vierten Ausführungsbeispiel,
- Fig. 6: einen Ausschnitt eines unwirksam gehaltenen Durchlässigkeitsbereiches gemäß einem fünften Ausführungsbeispiel,
- Fig. 7: ein sechstes Ausführungsbeispiel einer durch Schnitte hergestellten Perforierung,
- Fig. 8: ein siebtes Ausführungsbeispiel einer durch Schnitte hergestellten Perforierung,
- Fig. 9: ein achtes Ausführungsbeispiel einer durch Schnitte, hergestellten Perforierung.

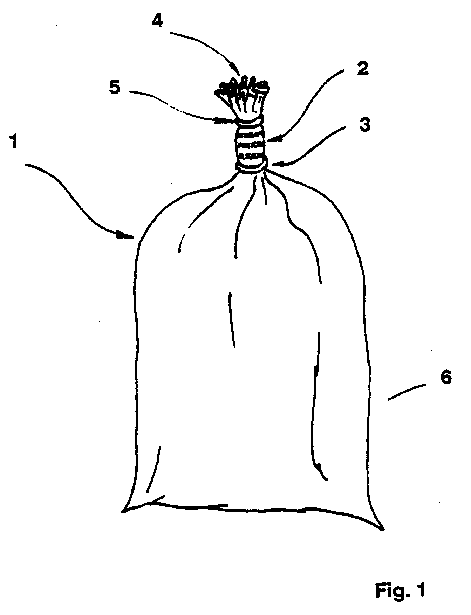

Gemäß Fig. 1 weist ein verschließbarer Behälter aus flüssigkeits- und gasundurchlässigem Wandmaterial 6 einen für Flüssigkeiten, Gase und Dämpfe durchlässigen Durchlässigkeitsbereich 2 auf (hier mit Strichlinien gekennzeichnet). Der Durchlässigkeitsbereich 2 wird durch die Sperreinrichtung 3 von dem Behälterinhalt getrennt und dadurch unwirksam gehalten. Eine Einbringöffnung 4 ist mit einer Verschlußeinrichtung 5 verschlossen. Die Verschlußeinrichtung 5 bleibt auch während eines Desinfektions- bzw. Sterilisationsverfahrens geschlossen. Der Durchlässigkeitsbereich 2 liegt zwischen der Verschlußeinrichtung 5 und der Sperreinrichtung 3 auf einem Faltenhals.

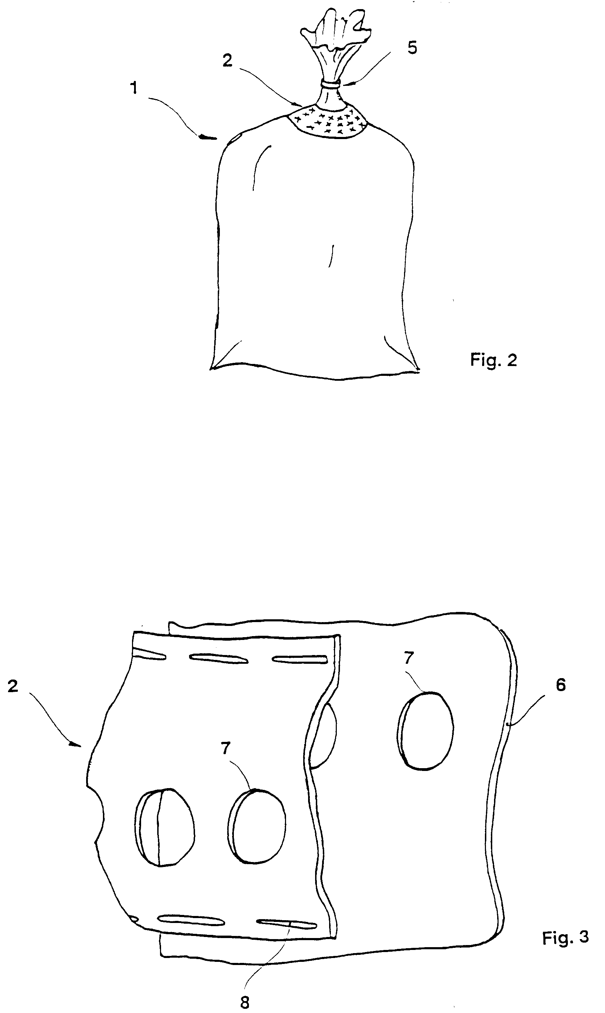

Fig. 2 zeigt einen mit der Verschlußeinrichtung 5 verschlossenen Behälter 1. Der Durchlässigkeitsbereich 2 steht mit dem Behälterinhalt in Verbindung. Der Durchlässigkeitsbereich 2 erstreckt sich als schmaler perforierter Streifen umlaufend auf der Behälterwand 6.

Fig. 3 zeigt einen Ausschnitt eines Durchlässigkeitsbereiches 2 mit Durchgangsöffnungen 7. Die Durchgangsöffnungen 7 sind derart zueinander versetzt angeordnet, daß kein geradliniger Verbindungsweg zwischen dem Inhalt des Behälters und seiner Umgebung besteht. Die zweite Folienlage ist durch Verbindungsstellen 8 dauerhaft mit der Behälterwandung 6 verbunden.

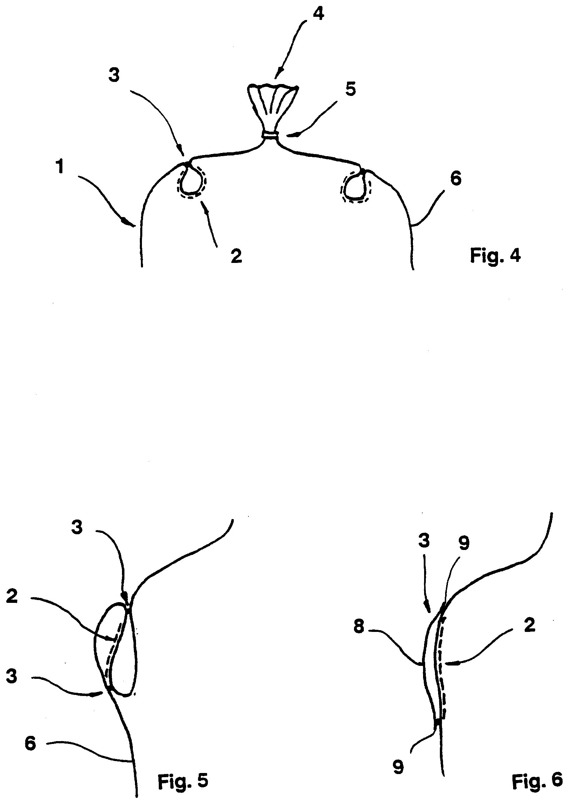

Fig. 4 zeigt ein Ausführungsbeispiel des Behälters mit einem durch eine Sperreinrichtung 3 unwirksam gehaltenen Durchlässigkeitsbereich 2 des Wandmateriales 6. Der Behälter ist im Bereich der Einbringöffnung 4 mit einer Verschlußeinrichtung 5 dauerhaft verschlossen. Bei der Sperreinrichtung 3 handelt es sich vorzugsweise um eine Verklebung mit geringer Temperaturfestigkeit.

Fig. 5 zeigt ein Ausführungsbeispiel zum Unwirksamhalten des Durchlässigkeitsbereiches durch Überfalten des Bereiches mit Wandmaterial 6 in Verbindung mit der Sperreinrichtung 3. Bei dieser Faltung wird der Durchlässigkeitsbereich 2 insbesondere auch während des Einbringens von Abfall vollständig verschlossen gehalten und von dem Abfall getrennt. Dadurch, daß der Durchlässigkeitsbereich 2 zunächst vollständig von der Umgebung und dem Inhalt des Behälters getrennt ist, ist es in vorteilhafter Weise möglich, den Durchlässigkeitsbereich 2 mit keimtötenden Mitteln oder Farbindikatoren zu versehen.

Fig. 6 zeigt ein Ausführungsbeispiel zum Unwirksamhalten des Durchlässigkeitsbereiches 2 mittels einer Sperreinrichtung 3. Die Sperreinrichtung besteht aus einer Folie 8 und gegebenenfalls aus einer Adhäsivmittelschicht 9. So ist es möglich, die Folie 8 aus einem vorzugsweise wärmeunbeständigen Stoff zu fertigen und/oder die Adhäsivmittelschicht derart auszulegen, daß sich diese bei Eintreten einer vorbestimmten Veränderung eines physikalischen Parameters der unmittelbaren Umgebung des Behälters löst.

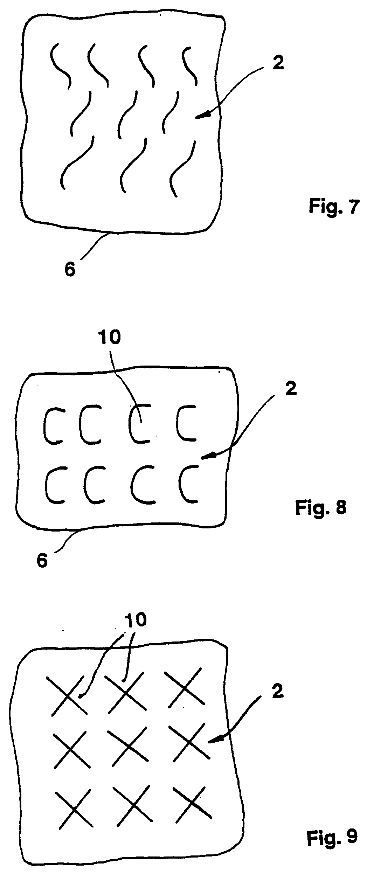

Fig. 7 zeigt einen Ausschnitt aus einem durch Schnitte erzeugten Durchlässigkeitsbereich 2 in dem Behälterwandmaterial 6.

Fig. 8 zeigt einen Ausschnitt aus einem ebenfalls durch Schnitte erzeugten Durchlässigkeitsbereich 2 in dem Behälterwandmaterial 6. Durch die gezeigte Schnittform entstehen klappbare Abschnitte 10, welche durch die Eigenelastizität des Materiales des Durchlässigkeitsbereiches in eine Stellung zurückfedern können, in welcher der Öffnungsquerschnitt der einzelnen Durchgänge verringert wird.

Fig. 9 zeigt einen Ausschnitt aus einem durch gekreuzte Schnitte erzeugten Durchlässigkeitsbereich 2. Auch in diesem Ausführungsbeispiel verringert sich der Öffnungsquerschnitt bei Beendigung eines Gas- bzw. Dampfaustausches selbständig durch aufgrund der Eigenelastizität des Materials des Durchlässigkeitsbereiches zurückfedernde Klappen 10.

Der vorstehend anhand von Ausführungsbeispielen beschriebene Behälter kann beispielsweise in folgender Weise eingesetzt werden: In einem Krankenhaus wird von einem Operationssaal kommender, kontaminierter Abfall in den, an einer ringförmigen Sackhalterung eingehängten und im Bereich der Einbringöffnung offen gehaltenen Behälter eingebracht. Nach Einbringen des kontaminierten Abfalles wird der gefüllte Behälter durch Bilden eines Faltenhalses im Bereich der Einbringöffnung mit einer unter Wärmeeinwirkung sich öffnenden Kunststoffschelle derart verschlossen, daß der Durchlässigkeitsbereich von dem Behälterinhalt getrennt und dadurch unwirksam gehalten ist. Dann wird der Behälter in einem Zwischenbereich des Faltenhaltes zwischen der Einbringöffnung und dem Durchlässigkeitsbereich mit einer dauerhaften Verschlußeinrichtung verschlossen. Der gefüllte Behälter wird verschlossen in einen Beschickungscontainer hineingestellt und darin in eine Vakuumdesinfektionskammer eingebracht. Die Desinfektionskammer wird geschlossen. Der Desinfektionskammer wird Heißdampf zugeleitet. Dieser erhitzt den Behälter und die wärmeunbeständige Kunststoffschelle. Die Kunststoffschelle löst sich und fällt ab. Die Verschlußeinrichtung ist hitzebeständig und hält die Einbringöffnung weiterhin geschlossen. Durch Lösen der Kunststoffschelle wird ein Verbindungsweg zwischen dem Durchlässigkeitsbereich und dem Inhalt des Behälters freigegeben. Die Heißdampfzufuhr wird eingestellt und der Druck in der Desinfektionskammer abgesenkt. Bei dieser Druckabsenkung entweichen Gase und Dämpfe über den Durchlässigkeitsbereich aus dem Inneren des Behälters. Die Druckabsenkung wird bis zum Erreichen eines bestimmten Unterdruckes durchgeführt. Die Druckabsenkung wird eingestellt und erneut Heißdampf der Desinfektionskammer zugeleitet. Der Heißdampf dringt durch den Durchlässigkeitsbereich in den Behälter ein und erhitzt den Inhalt des Behälters. Aufgrund des Zuströmens des Heißdampfes erfolgt ein Druckanstieg in der Desinfektionskammer. Nach Ablauf einer bestimmten Haltezeit bzw. Heißdampfeinwirkungszeit erfolgt eine erneute Druckabsenkung mit einem erneuten Gas- bzw. Dampfaustritt aus dem Inneren des Behälters über den Durchlässigkeitsbereich. Druckabsenkung und Heißdampfzutritt können mehrmals mit auch veränderten verfahrensspezifischen Parametern, insbesondere Dampftemperatur, Einwirkungszeit und Unterdruck, mehrfach ausgeführt werden. Nach Beendigung des Desinfektionsverfahrens wird die Desinfektionskammer geöffnet, der Beschickungscontainer der Desinfektionskammer entnommen und der Inhalt des Beschickungscontainers in einen Preßcontainer gekippt. Der Inhalt des Preßcontainers wird einer Deponie oder einer Verbrennungsanlage zugeführt.

Die Erfindung ist nicht auf das vorstehend beschriebene Ausführungsbeispiel beschränkt. Beispielsweise kann der Durchlässigkeitsbereich auch durch eine wärmeunbeständige Folie bis zur Durchfürung einer Sterilisation oder Desinfektion verschlossen werden.

## Patentansprüche

1. Verschließbarer sack- bzw. beutelartiger Behälter (1) für infektiösen Abfall, mit flüssigkeits- und gasundurchlässigem flexiblem Wandmaterial (6) und mindestens einem darin ausgebildeten, für Flüssigkeiten, Gase und Dämpfe durchlässigen Durchlässigkeitsbereich (2), und einer Einbringöffnung (4), welche nach dem Einbringen von Abfall geschlossen wird, **dadurch gekennzeichnet,** daß eine Sperreinrichtung (3) vorgesehen ist, welche den Durchlässigkeitsbereich (2) bis zur Veränderung eines physikalischen Parameters der unmittelbaren Umgebung des Behälters (1) durch einen Desinfektions- oder Sterilisationsprozeß um einen vorbestimmten Betrag von dem Behälterinhalt trennt.

2. Verschließbarer sack- bzw. beutelartiger Behälter (1) für infektiösen Abfall, mit flüssigkeits- und gasundurchlässigem flexiblem Wandmaterial (6) und mindestens einem darin ausgebildeten, für Flüssigkeiten, Gase und Dämpfe durchlässigen Durchlässigkeitsbereich (2), und einer Einbringöffnung (4), welche nach dem Einbringen von Abfall geschlossen wird, **dadurch gekennzeichnet,** daß eine Sperreinrichtung (3) vorgesehen ist, welche den Durchlässigkeitsbereich (2) bis zur Veränderung eines physikalischen Parameters der unmittelbaren Umgebung des Behälters (1) durch einen Desinfektions- oder Sterilisationsprozeß um einen vorbestimmten Betrag abdeckt.

3. Behälter (1) nach einem der Ansprüche 1 oder 2, **dadurch gekennzeichnet,** daß mit Eintreten der Veränderung des physikalischen Parameters um den vorbestimmten Betrag die Sperrvorrichtung (3) eine Verbindung zwischen dem Inhalt des Behälters (1) und dessen Umgebung über den Durchlässigkeitsbereich (2) freigibt.

4. Behälter (1) nach mindestens einem der Ansprüche 1 bis 3, **dadurch gekennzeichnet,** daß der Durchlässigkeitsbereich (2) durch Perforation bzw. Nadeln, Stanzen und/oder Schneiden des Behälterwandmateriales (6) gebildet ist.

5. Behälter (1) nach mindestens einem der Ansprüche 1 bis 4, **dadurch gekennzeichnet,** daß der Durchlässigkeitsbereich (2) durch Substitution von Wandmaterial mit mindestens einem flüssigkeits-, gas- und dampfdurchlässigen Element gebildet ist.

6. Behälter (1) nach mindestens einem der Ansprüche 1 bis 5, **dadurch gekennzeichnet,** daß der Durchlässigkeitsbereich (2) aus einem mindestens zweilagig ausgebildeten folienartigen Element besteht, dessen Lagen mit Durchgangsöffnungen (7) versehen sind, welche derart zueinander versetzt angeordnet sind, daß jede Öffnung zumindest teilweise von einer Folienlage überdeckt wird.

7. Behälter (1) nach mindestens einem der Ansprüche 1 bis 6, **dadurch gekennzeichnet,** daß der Durchlässigkeitsbereich (2) streifenförmig ausgebildet ist.

8. Behälter nach Anspruch 7, **dadurch gekennzeichnet,** daß der Durchlässigkeitsbereich (2) sich zumindest um einen Teil des Umfanges der Behälterwand erstreckt.

9. Behälter (1) nach mindestens einem der Ansprüche 2 bis 8, **dadurch gekennzeichnet,** daß die Sperrvorrichtung (3) als auf den Durchlässigkeitsbereich (2) aufgebrachte Folie (8) ausgebildet ist.

10. Behälter (1) nach mindestens einem der Ansprüche 1 bis 9, **dadurch gekennzeichnet,** daß die Sperrvorrichtung (3), insbesondere die Folie (8), auf ihrer dem Durchlässigkeitsbereich (2) zugewandten Seite eine Adhäsivmittelschicht (9) aufweist.

11. Behälter (1) nach Anspruch 10, **dadurch gekennzeichnet,** daß die Adhäsivmittelschicht (9) aus einer wärmeunbeständigen Klebeschicht besteht.

12. Behälter (1) nach mindestens einem der Ansprüche 1 bis 11, **dadurch gekennzeichnet,** daß eine Verschlußeinrichtung (5) vorgesehen ist, mittels derer der Behälter im Bereich der Einbringöffnung (4) verschließbar ist und während eines Desinfektions- bzw. Sterilisationsverfahrens geschlossen bleibt.

13. Behälter (1) nach Anspruch 1, **dadurch gekennzeichnet,** daß die Sperreinrichtung (3) in Schließstellung den Behälterinhalt von dem Durchlässigkeitsbereich (2) durch ein Zusammenschnüren des beutelartigen Behälters (1) im Bereich der Einbringöffnung (4) trennt.

14. Behälter (1) nach mindestens einem der Ansprüche 1 bis 13, **dadurch gekennzeichnet,** daß an dem Behälter (1) zum Zwekke des Verschließens ein Faltenhals bildbar ist und der Durchlässigkeitsbereich (2) im Faltenhals zwischen der Sperrvorrichtung (3) und der Verschlußeinrichtung (5) liegt.

15. Behälter (1) nach mindestens einem der Ansprüche 1 bis 14, **dadurch gekennzeichnet,** daß die Sperreinrichtung (3) in Schließstellung den beutelartigen Behälter (1) durch Zusammenschnüren des Faltenhalses verschlossen hält und sich bei Auftreten eines vorbestimmten Temperatursprunges öffnet, wobei der vormals zusammengeschnürte Bereich des Faltenhalses einen Durchgangsquerschnitt freigibt und eine Verbindung zwischen dem Durchlässigkeitsbereich (2) und dem Behälterinhalt entsteht.

16. Behälter (1) nach mindestens einem der Ansprüche 1 bis 15, **dadurch gekennzeichnet,** daß die Sperreinrichtung (3) als Schlinge oder Ring ausgebildet ist, welcher mit mindestens einer Sollbruchstelle versehen ist.

17. Behälter (1) nach mindestens einem der Ansprüche 1 bis 16, **dadurch gekennzeichnet,** daß die Sperreinrichtung (3) in Schließstellung den beutelartigen Behälter (1) durch Zusammenschnüren des Faltenhalses derart verschlossen hält, daß eine Verbindung zwischen dem Durchlässigkeitsbereich (2) und dem Inhalt des Behälters (1) zunächst gesperrt ist und bei Verformung des Behälters (1) durch Auftreten eines vorbestimmten Überdrucks im Behälterinneren freigegeben wird, so daß der Durchlässigkeitsbereich (2) mit dem Inhalt des Behälters (1) in Verbindung steht.

18. Verschließbarer sack- bzw. beutelartiger Behälter (1) für infektiösen Abfall, mit flüssigkeits- und gasundurchlässigem flexiblem Wandmaterial (6) und mindestens einem darin ausgebildeten, für Flüssigkeiten, Gase und Dämpfe durchlässigen Durchlässigkeitsbereich (2), und einer Einbringöffnung (4), welche nach dem Einbringen von Abfall geschlossen wird, **dadurch gekennzeichnet,** daß der Durchlässigkeitsbereich (2) durch Überfalten mit einem undurchlässigen Wandbereich zunächst verdeckt, und durch eine Sperreinrichtung (3), welche den Durchlässigkeitsbereich (2) bis zur Veränderung eines physikalischen Parameters der unmittelbaren Umgebung des Behälters durch einen Desinfektions-oder Sterilisationsprozeß unwirksam hält, vorzugsweise einer Verklebung mit geringer Temperaturfestigkeit, in einer Schließstellung flüssigkeits-, gas- und dampfdicht gehalten ist.

## Claims

1. A closable, sack- or bag-like container (1) for infectious waste, with flexible wall material (6), which is impermeable to liquid and gas, and at least one permeable region (2) which is provided within the wall material (6) and is permeable to liquids, gases and vapours, and an filling opening (4) which is to be closed after filling with waste, **characterized in that** a blocking means (3) is provided which separates the permeable region (2) from the container contents until a physical parameter in the immediate environment of the container (1) changes as a result of a disinfection or sterilisation process.

2. A closable sack- or bag-like container (1) for infectious waste, with flexible wall material (6), which is impermeable to liquid and gas, and at least one permeable region (2) which is provided within the wall material (6) and is permeable to liquids, gases and vapours, and a filling opening (4) which is closed after filling with waste, **characterized in that** a blocking means (3) is provided which covers the permeable region (2) until a physical parameter in the immediate environment of the container (1) changes by a given degree as a result of a disinfection or sterilisation process.

3. A container (1) according to one of claims 1 or 2, **characterized in that** with the change in the physical parameter by the given degree, the blocking means (3) releases a connection between the contents of the container (1) and its environment via the permeable region (2).

4. A container (1) according to at least one of claims 1 to 3, **characterized in that** the permeable region (2) is formed by perforating or piercing, punching and/or cutting the container wall material (6).

5. A container (1) according to at least one of claims 1 to 4, **characterized in that** the permeable region (2) is formed by substituting the wall material with at least one liquid-, gas- and vapour-permeable element.

6. A container (1) according to at least one of claims 1 to 5, **characterized in that** the permeable region (2) is formed by a film-like element comprising at least two layers, which are provided with through openings (7) offset relative to one another in such a manner that each opening is at least partially covered by a film layer.

7. A container (1) according to at least one of claims 1 to 6, **characterized in that** the permeable region (2) is constructed in the form of a strip.

8. A container according to claim 7, **characterized in that** the permeable region (2) extends over at least part of the circumference of the container wall.

9. A container (1) according to at least one of claims 2 to 8, **characterized in that** the blocking device (3) is constructed as a film (8) fitted over the permeable region (2).

10. A container (1) according to at least one of claims 1 to 9, **characterized in that** the blocking means (3) more particularly the film (8) comprises an adhesive layer (9) on its side facing the permeable region (2).

11. A container (1) according to claim 10, **characterized in that** the adhesive layer (9) is formed by a layer of adhesive which is non-resistant to heat.

12. A container (1) according to at least one of claims 1 to 11, **characterized in that** a closing device (5) is provided by means of which the container is closable in the region of the filling opening (4) and remains closed during a disinfection or sterilisation process.

13. A container (1) according to claim 1, **characterized in that** in the closed position the blocking means (3) separates the container contents from the permeable region (2) by constricting the bag-like container (1) in the region of the filling opening (4).

14. A container (1) according to at least one of claims 1 to 13, **characterized in that** a pleated neck can be formed on the container (1) for the purpose of closure and the permeable region (2) lies in the pleated neck between the blocking means (3) and the closing device (5).

15. A container (1) according to at least one of claims 1 to 14, **characterized in that** in the closed position the blocking means (3) holds the bag-like container (1) closed by constricting the pleated neck and opens in the event of a predetermined jump in temperature, the previously constricted region of the pleated neck releasing an open cross section and a connection being formed between the permeable region (2) and the container contents.

16. A container (1) according to at least one of claims 1 to 15, **characterized in that** the blocking means (3) is constructed as a loop or ring, which is provided with at least one intended breakage point.

17. A container (1) according to at least one of claims 1 to 16, **characterized in that** in the closed position the blocking means (3) holds the bag-like container (1) closed by constricting the pleated neck in such a manner that a connection between the permeable region (2) and the contents of the container (1) is initially blocked and is released with the deformation of the container (1) caused by the presence of a given excess pressure in the container interior, so that the permeable region (2) is connected to the contents of the container (1).

18. A closable, sack- or bag-like container (1) for infectious waste, with flexible wall material (6), which is impermeable to liquid and gas, and at least one permeable region (2) which is provided within the wall material (6) and is permeable to liquids, gases and vapours, and a filling opening (4) which is closed after filling with waste, **characterized in that** the permeable region (2) is firstly covered by folding a non-permeable wall region thereover, and is held in a closed position in a liquid-, gas- and vapour-tight manner by a blocking means (3), preferably and adhesive means with low temperature resistance, which renders the permeable region (2) effective until a change in a physical parameter in the immediate environment of the container is brought about by a disinfection or sterilisation process.

## Revendications

1. Récipient (1) pouvant être fermé, sous la forme d'un sac ou d'un sachet, pour des déchets infectieux, dont le matériau de paroi (6) est flexible, imperméable au fluide et au gaz et dans lequel sont ménagées au moins une zone perméable (2) laissant passer les gaz, les vapeurs et les fluides et une ouverture d'admission (4) qui est fermée après l'admission des déchets, caractérisé en ce qu'il est prévu un dispositif d'obturation (3) qui sépare la zone perméable (2) du contenu du récipient, jusqu'à la modification d'un paramètre physique de l'environnement direct du récipient (1) d'une valeur prédéterminée par un processus de stérilisation ou de désinfection.

2. Récipient (1) pouvant être fermé, sous la forme d'un sac ou d'un sachet, pour des déchets infectieux, dont le matériau de paroi (6) est flexible, imperméable au fluide et au gaz et dans lequel sont ménagées au moins une zone perméable (2) laissant passer les gaz, les vapeurs et les fluides et une ouverture d'admission (4) qui est fermée après l'admission des déchets, caractérisé en ce qu'il est prévu un dispositif d'obturation (3) qui recouvre, la zone perméable (2), jusqu'à la modification d'un paramètre physique de l'environnement direct du récipient (1) d'une valeur prédéterminée par un processus de stérilisation et de désinfection.

3. Récipient (1) selon l'une des revendications 1 ou 2, caractérisé en ce le paramètre physique étant modifié de la valeur prédéterminée, le dispositif d'obturation (3) libère une connexion entre le contenu du récipient (1) et son environnement, par l'intermédiaire de la zone perméable (2).

4. Récipient (1) selon au moins l'une des revendications 1 à 3, caractérisé en ce que la zone perméable (2) est formée de perforations par piqûres, par poinçonnage et/ou découpe du matériau (6) de la paroi du récipient.

5. Récipient (1) selon au moins l'une des revendications 1 à 4, caractérisé en ce que la zone perméable (2) est constituée par substitution de la matière de paroi avec au moins un élément perméable au liquide, au gaz et à la vapeur.

6. Récipient (1) selon au moins l'une des revendications 1 à 5, caractérisé en ce que la zone perméable (2) se compose d'un élément en forme de feuille et comportant au moins deux couches, dont les couches sont munies d'ouvertures de passage (7) qui sont disposées de manière décalée les unes par rapport aux autres de manière que chaque ouverture soit du moins partiellement recouverte par une couche de feuille.

7. Récipient (1) selon au moins l'une des revendications 1 à 6, caractérisé en ce que la zone perméable (2) est striée.

8. Récipient (1) selon la revendication 7, la zone perméable (2) s'étend sur au moins une partie de la périphérie de la paroi de récipient.

9. Récipient (1) selon au moins l'une des revendications 2 à 8, caractérisé en ce que le dispositif d'obturation (3) se présente comme une feuille (8) appliquée sur la zone perméable (2).

10. Récipient (1) selon au moins l'une des revendications 1 à 9, caractérisé en ce que le dispositif d'obturation (3), en particulier la feuille (8), présente, sur sa face tournée vers la zone perméable (2), une couche intermédiaire adhésive (9).

11. Récipient (1) selon la revendication 10, caractérisé en ce que la couche intermédiaire adhésive (9) se compose d'un revêtement de colle thermosensible.

12. Récipient (1) selon au moins l'une des revendications 1 à 11, caractérisé en ce qu'il est prévu un dispositif de fermeture (5) au moyen duquel peut être fermé le récipient dans la zone de l'ouverture d'admission (4) et reste fermé pendant un processus de stérilisation ou de désinfection.

13. Récipient (1) selon la revendication 1, caractérisé en ce que le dispositif d'obturation (3) sépare, en position de fermeture, le contenu du récipient de la zone perméable (2), par ficelage du récipient en forme de sachet (1), dans la zone de l'ouverture d'admission (4).

14. Récipient (1) selon au moins l'une des revendications 1 à 13, caractérisé en ce que, pour fermer le récipient (1), on peut former un col à soufflet et en ce que la zone perméable (2) se trouve dans le col à soufflet entre le dispositif d'obturation (3) et le dispositif de fermeture (5).

15. Récipient (1) selon au moins l'une des revendications 1 à 14, caractérisé en ce que le dispositif d'obturation (3), en position fermée, maintient le récipient en forme de sachet (1) fermé par ficelage du col à soufflet et s'ouvre quand survient un saut de température prédéterminé, la zone, ficelée auparavant, du col à soufflet libère ainsi une section de passage et une connexion se crée entre la zone perméable (2) et le contenu du récipient.

16. Récipient (1) selon au moins l'une des revendications 1 à 15, caractérisé en ce que le dispositif d'obturation (3) revêt la forme d'une boucle ou d'un anneau qui est muni d'au moins un point de rupture.

17. Récipient (1) selon au moins l'une des revendications 1 à 16, caractérisé en ce que le dispositif d'obturation (3), en position de fermeture, maintient, par ficelage du col à soufflet, le récipient en forme de sachet (1) fermé, de telle sorte qu'une connexion entre la zone perméable (2) et le contenu du récipient (1) soit obturée puis libérée, au moment de la déformation du récipient (1), par la génération d'une surpression prédéterminée à l'intérieur du récipient, si bien que la zone perméable (2) soit en connexion avec le contenu du récipient (1).

18. Récipient (1) pouvant être fermé, sous la forme d'un sac ou d'un sachet pour des déchets infectieux, dont le matériau de paroi (6) est flexible, imperméable au fluide et au gaz et dans lequel sont ménagées au moins une zone perméable (2) laissant passer les gaz, les vapeurs et les fluides et une ouverture d'admission (4) qui est fermée après l'admission des déchets, caractérisé en ce que la zone perméable (2) est tout d'abord recouverte par un repliage avec une zone de paroi imperméable et en ce qu'elle est maintenue en position fermée étanche au gaz, au liquide et au fluide par un dispositif d'obturation (3), de préférence un collage de faible résistance thermique, qui maintient la zone perméable (2) inactive jusqu'à la modification d'un paramètre physique de l'environnement direct du récipient par un processus de désinfection ou de stérilisation.
